# EUROPEAN PATENT SPECIFICATION

(11) **EP 2 658 485 B1**
(45) Date of publication and mention of the grant of the patent: **26.10.2016**
(21) Application number: 11767518.1
(22) Date of filing: 22.09.2011
(51) Int. Cl.: A61F 2/91, A61F 2/915

(54) **STENT**
STENT
ENDOPROTHÈSE

(30) Priority: 28.12.2010 US 201061427689 P
(43) Date of publication of application: 06.11.2013
(73) Proprietor: Boston Scientific Scimed, Inc., Maple Grove, MN 55311-1566 (US)
(72) Inventor: MEYER, Michael P., Richfield, Minnesota 55423 (US); THESINGH, Doug, Minneapolis, Minnesota 55406-2314 (US); PATRAS, Sandy, Loretto, Minnesota 55357 (US); JULSON, Alison, Ramsey, Minnesota 55303 (US)
(74) Representative: Peterreins Schley
(86) International application number: PCT/US2011/052720
(87) International publication number: WO 2012/091770

(56) References cited:
- EP-A2- 1 719 479
- WO-A1-97/25937
- WO-A1-2007/131798
- US-A1- 2004 243 216

## Description

### BACKGROUND OF THE INVENTION

### 2. Description of the Related Art

A stent is a medical device introduced to a body lumen and is well known in the art. Typically, a stent is implanted in a blood vessel at the site of a stenosis or aneurysm endoluminally, i.e. by so-called "minimally invasive techniques" in which the stent in a radially reduced configuration, optionally restrained in a radially compressed configuration by a sheath and/or catheter, is delivered by a stent delivery system or "introducer" to the site where it is required. The introducer may enter the body from an access location outside the body, such as through the patient's skin, or by a "cut down" technique in which the entry blood vessel is exposed by minor surgical means.

Stents, grafts, stent-grafts, vena cava filters and, more generally, expandable medical frameworks, and similar implantable medical devices, are radially expandable endoprostheses, which are typically intravascular implants capable of being implanted transluminally and enlarged radially after being introduced percutaneously. Stents, grafts, stent grafts and other expandable medical frameworks, may be implanted in a variety of body lumens or vessels, such as within the vascular system, urinary tracts, bile ducts, fallopian tubes, coronary vessels, secondary vessels, etc. These devices may be used for a variety of purposes including to reinforce body vessels and to prevent restenosis following angioplasty in the vascular system. They may be self- expanding, expanded by an internal radial force, such as when mounted on a balloon, or a combination of self-expanding and balloon expandable (hybrid expandable).

Any of the above-mentioned devices may be created by methods including cutting or etching a design from a tubular stock or from a flat sheet. The flat sheet may subsequently be rolled.

They may also be made from one or more interwoven wires or braids.

The art referred to and/or described herein is not intended to constitute an admission that any patent, publication or other information referred to herein is "prior art" with respect to this invention.

Without limiting the scope of the invention a brief summary of some of the claimed embodiments of the invention is set forth below. Additional details of the summarized embodiments of the invention and/or additional embodiments of the invention may be found in the Detailed Description of the Invention below.

Published international patent application no. WO 2007/131798 A1 discloses a stent exhibiting a succession of turns around the axis, adjacent turns being connected by connector portions distributed around the circumference of the prosthesis, each of the turns being made up of struts that extend the length of the turn and are interspersed by inflection zones located at the axial ends of each of the turns. When the prosthesis expands radially, gaps open up between adjacent struts of each of the turns, the inflection zones being distributed regularly around the circumference so that the gaps are substantially the same size as each other around the circumference of the turn, at least every second turn exhibiting a stagger zone within which the said gap between adjacent said struts is of an individual size different from that common to the other gaps of that second turn.

### BRIEF SUMMARY OF THE INVENTION

The present invention relates to an expandable medical framework as set out in claim 1. Other embodiments are described in the dependent claims.

These and other embodiments which characterize the invention are pointed out with particularity in the claims annexed hereto and forming a part hereof. However, for further understanding of the invention, its advantages and objectives obtained by its use, reference should be made to the drawings which form a further part hereof and the accompanying descriptive matter, in which there are illustrated and described further embodiments of the invention.

### BRIEF DESCRIPTION OF THE SEVERAL VIEWS OF THE DRAWING(S)

A detailed description of the invention is hereafter described with specific reference being made to the drawings.
FIG. 1 a shows a side view of an expandable medical framework in the form of a crimped stent.
FIG. 1b shows a side view of an expandable medical framework in the form of a stent as cut.
FIG. 1c shows a side view of an expandable medical framework in the form of a stent in an expanded state.
FIG. 2 shows a flat view of an inventive expandable medical framework in the form of a stent as cut.
FIG. 3 shows a flat view of an inventive expandable medical framework in the form of a stent as cut.
FIG. 4 shows a flat view of an inventive expandable medical framework in the form of a stent as cut.
FIG. 5 shows a side view of small portion of an expandable medical stent.
FIG. 6 shows a cross-section of the stent of FIG. 5 taken along 6-6.
FIG. 7 shows an individual strut of the expandable medical framework of FIGS 2-4.
FIG. 8 is a cross-sectional view of a catheter carrying an expandable medical device in the form of a stent.
FIG. 8A shows an enlarged view of the distal end of the catheter of FIG. 8.

### DETAILED DESCRIPTION OF THE INVENTION

While this invention may be embodied in many different forms, there are described in detail herein specific embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

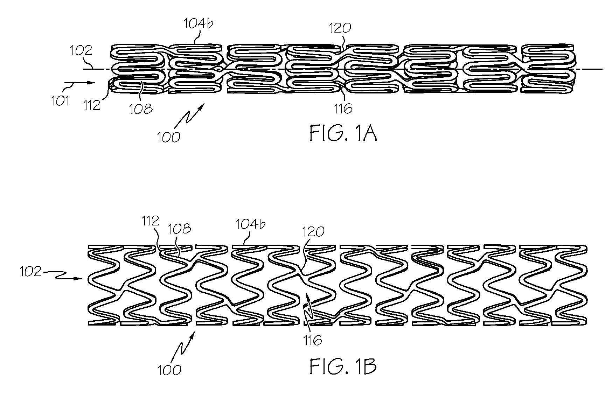

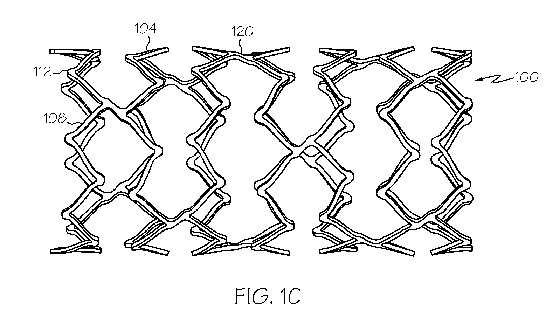

In one aspect, the invention is directed to an expandable medical framework, shown at 100 in Fig. 1a, for implantation in a mammalian body. Typically, the framework will be sized and configured for implantation in the human body. The expandable medical framework, is in the form of a tube with a flowpath 101 therethrough. Longitudinal axis 102 extends through the tube. As shown in Fig. 1a the expandable medical framework, in the form of a stent, is in a crimped state. Fig. 1b depicts an inventive expandable medical framework, in the form of a stent as cut. Fig. 1c depicts an inventive expandable medical framework, in the form of an expanded stent.

As shown in a crimped state in Fig. 1a, and in an as-cut state in Figs, 1b, and 2-4, and in an expanded state in Fig. 1c, expandable medical framework 100, in the form of a stent, comprises a plurality of serpentine bands 104 including end serpentine bands 104a and intermediate serpentine bands 104b. Intermediate serpentine bands 104b are disposed between end serpentine bands 104a. Each serpentine band 104 comprises a plurality of alternating band struts 108 and turns 1 12. Desirably band struts 108 are straight. Turns 1 12 desirably are rounded. In other embodiments, the turns may be generally elliptical, square or pointed or any other configuration.

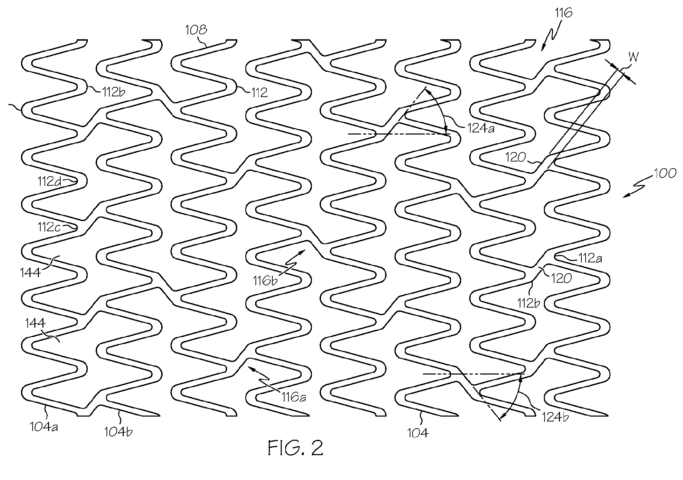

As shown at least in Fig. 2, turns 112 include proximal turns 1 12a which are located at a proximal end of a serpentine band 104 and distal turns 112b which are located at a distal end of a serpentine band 104. The proximal turns may also be referred to as troughs or valleys. The distal turns may also be referred to as peaks.

Expandable medical framework 100 also comprises a plurality of connector columns 1 16. Each connector column 1 16 comprises a plurality of connector struts 120. Each connector column 116 is disposed between and connects two serpentine bands 104. Each connector strut 120 is connected at one end to a turn 1 12, typically a proximal turn 1 12a of one serpentine band 104 and connected at the other end to a turn 112, typically a distal turn 112b of another serpentine band 104.

The turns 112 of a serpentine band of the expandable medical framework comprise connected turns 1 12c and unconnected turns 1 12d. Each connected turn 1 12c has a connector strut 120 extending therefrom. Each unconnected turn does not have any connector struts extending therefrom. The only struts extending from an unconnected turn are the two band struts which are joined by the unconnected turn. Connected turns may be located at a proximal end of a serpentine band, at a distal end of a serpentine band or at both the proximal and distal ends of a serpentine band.

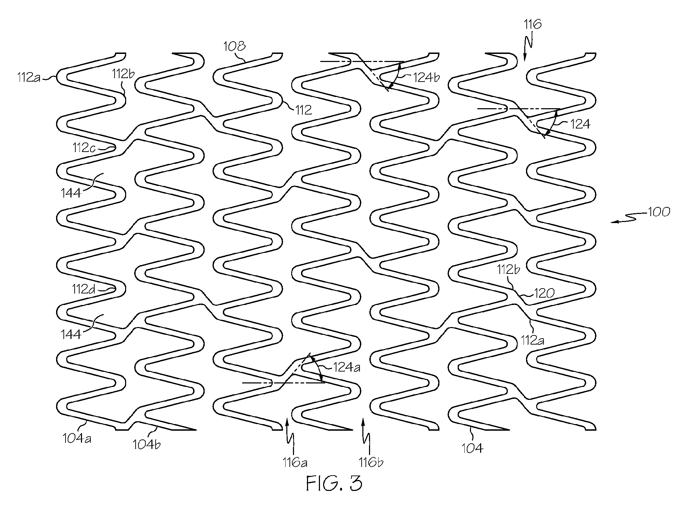

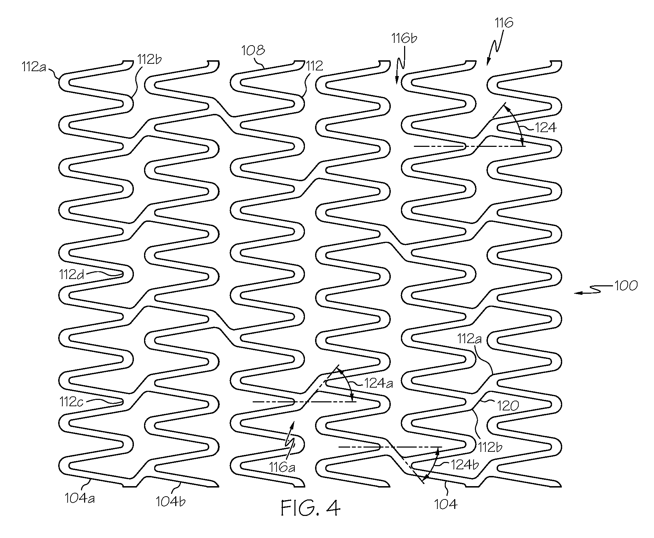

Each of the end serpentine bands 104a is connected to one of the intermediate serpentine bands 104b via four or five connector struts 120 which are equally spaced about the circumference of the expandable medical framework. Figs. 2 and 3 show an expandable medical framework with four connector struts connecting the two end-most serpentine bands at either end of the stent. Fig. 4 shows an expandable medical framework with five connector struts connecting the two end-most serpentine bands at either end of the stent.

As shown in Figures 2-4, each two adjacent intermediate serpentine bands 104b is connected one to the other via two connector struts 120 which are equally spaced about the circumference of the expandable medical framework.

Desirably, the ratio of the width W shown in Fig. 2, of the connector strut 120, as measured along the connector strut 120 in a non-radial direction normal to the length of the connector strut 120, to the width of the band strut 108, as measured along the band strut 108 in a non-radial direction normal to the length of the band strut 108, will range from 1.1 to 1.4.

Each turn 1 12 has a width, which may be measured along a longitudinal line bisecting the turn 112. Desirably, the ratio of the width of a turn 1 12 to the width of a band strut 108 is 1.6 to 1.8.

As further shown in Figures 2-4, connector struts 120 within a connector column are all oriented at the same angle 124 relative to a longitudinal axis 126 bisecting two connected struts 108. Connector struts 120 in adjacent connector columns 116 are oppositely oriented.

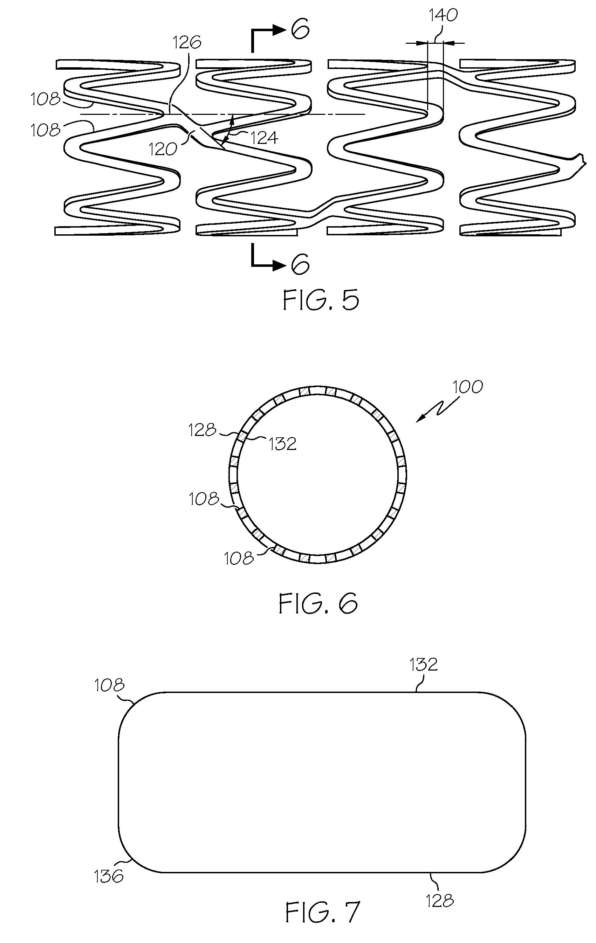

Thus, angle 124a of connector struts 120 in connector column 116a and angle 124b of connector struts 120 in connector column 116b are identical, however, they point in opposite directions relative to the longitudinal axis bisecting the connected turn from which the connector strut extends. Typically, this angle will be between 35° and 50° relative to a longitudinal axis bisecting the connected turn from which the connector strut extends. Angle 124 is also depicted in Fig. 5.

Because Fig. 5 is a side view, only one of the two connectors between adjacent serpentine bands is visible.

Typically, as shown in Figures 6 and 7, band struts 108 have outer surfaces 128 with rounded edges 136. The outer surface is the surface which points radially outward. The inner surface 132 faces radially inward. The band struts in 108 are straight. Desirably, other than the rounding of the edges of the band strut and any curvature inherent in the tubular nature of the stent, there is no other curvature to the band strut.

Typically, unconnected turns 1 12 each have an outer radius 140 as measured on the outside of the unconnected turn 112 of between 0.0035 inches and 0.0050 inches (0.089 mm and 0.127 mm).

The expandable medical framework will, desirably, have a thickness, as measured in a radial direction, of between 0.0025 inches and 0.0034 inches (0.0635 mm and 0.08636 mm).

In the expandable medical framework of Figures 2 and 3, each serpentine band has sixteen band struts 108. As previously discussed, two connector struts 120 connect intermediate serpentine bands 104b and four connector struts 120 connect the end serpentine bands 104a and an intermediate serpentine band 104b. This arrangement results in only three turns 1 12 of a serpentine band 104 being disposed between adjacent connector struts 120 connecting an end serpentine band 104a to an intermediate serpentine band 104b. This amounts to one peak and two troughs on one of the connected serpentine bands and two peaks and one trough on the other of the two serpentine bands.

This arrangement also results in only seven turns 1 12 of a serpentine band 104 being disposed between adjacent connector struts 120 connecting an intermediate serpentine band 104b to an adjacent intermediate serpentine band 104b. This amounts to three peaks and four troughs on one of the connected serpentine bands and four peaks and three troughs on the other of the two serpentine bands.

The four connector struts 120 between the end serpentine bands 104a and the intermediate serpentine bands 104b are equally spaced about the circumference of the expandable medical framework. Each interconnected intermediate serpentine band and end serpentine band has four equally sized cells 144 disposed about the
circumference of the stent. Similarly, the two connector struts 120 between the intermediate serpentine bands 104b are equally spaced about the circumference of the expandable medical framework. Each two interconnected intermediate serpentine bands has two equally sized cells 144 disposed about the circumference of the stent.

In the expandable medical framework of Fig. 4, each serpentine band has twenty band struts 108. As previously discussed, two connector struts 120 connect intermediate serpentine bands 104b and five connector struts 120 connect the end serpentine bands 104a and an intermediate serpentine band 104b. This arrangement results in only three turns 1 12 of a serpentine band being disposed between adjacent connector struts 120 connecting an end serpentine band 104a to an intermediate serpentine band 104b. This amounts to one peak and two troughs on one of the connected serpentine bands and two peaks and one trough on the other of the two serpentine bands.

This arrangement also results in only nine turns 112 of a serpentine band being disposed between adjacent connector struts 120 connecting an intermediate serpentine band 104b to an adjacent intermediate serpentine band 104b. This amounts to four peaks and five troughs on one of the connected serpentine bands and five peaks and four troughs on the other of the two serpentine bands.

The five connector struts 120 between the end serpentine bands 104a and the intermediate serpentine bands 104b are equally spaced about the circumference of the expandable medical framework. Each interconnected intermediate serpentine band and end serpentine band has five equally sized cells 144 disposed about the
circumference of the stent.

Similarly, the two connector struts 120 between the intermediate serpentine bands 104b are equally spaced about the circumference of the expandable medical framework. Each two interconnected intermediate serpentine bands have two equally sized cells 144 disposed about the circumference of the stent.

The expandable medical frameworks can have any suitable number of serpentine bands. A serpentine band can span any suitable distance along the length of the expandable framework. Similarly, a connector strut can span any suitable distance along the length of the expandable framework.

The expandable medical framework of Figures 2-4 is shown in the as cut state of the stent 100. In use, the stents may be provided in a reduced diameter configuration carried by a catheter. When provided in such a configuration such, as by crimping the stent, as shown by way of example in Fig. 1a, the band struts 108 of the stent may optionally extend parallel to the longitudinal axis 102 of the stent or close to parallel to the longitudinal axis 102 of the stent. The angle formed between adjacent band struts will be reduced compared to the angle in the as cut configuration. When in the fully expanded configuration, the angle formed between adjacent band struts may be the same as, greater than or less than that shown in Figs. 2-4.

The expandable medical frameworks can be of uniform outer and/or inner diameter or they can be provided with a variable outer and/or inner diameter. For example, the outer and/or inner diameters can include a taper at one or both ends of the framework. Optionally, one end or both ends can have a larger outer and/or inner diameter than the middle of the expandable medical framework. Optionally, one end or both ends can have a smaller outer and/or inner diameter than the middle of the expandable medical framework.

The inventive expandable medical frameworks are depicted as having a single flowpath extending therethrough. However, the inventive expandable medical frameworks may also be used as a part of a bifurcated medical framework such as, but not limited to, a bifurcated stent. Any of the expandable medical frameworks can serve as a branch of or as main body portion of a bifurcated expandable medical framework in general and a bifurcated stent in particular. It is within the scope of the invention for the expandable medical frameworks disclosed herein to be balloon-expandable or otherwise mechanically expandable, self-expanding or a combination of balloon expandable and/or
mechanically expandable and self-expanding. Thus, stents, grafts, stent-grafts, vena cava filters and other medical devices made in accordance with the invention may be balloon-expandable or otherwise mechanically expandable, self-expanding or a combination of balloon expandable and/or mechanically expandable and self-expanding. Desirably, they will be balloon expandable.

Any of the inventive expandable medical frameworks disclosed herein may be made from any suitable biocompatible materials including one or more polymers, one or more metals or combinations of polymer(s) and metal(s). Examples of suitable materials include biodegradable materials that are also biocompatible.

The expandable medical frameworks can have one or more components constructed from one or more metals, polymers or combinations thereof that are corrodible so as to dissolve, dissociate or otherwise break down in the body without ill effect. Examples of such materials have been referred to as being degradable, biodegradable, biologically degradable, erodable, bioabsorbable, bioresorbable, and the like. Biodegradable material will generally undergo breakdown or decomposition into harmless compounds as part of a normal biological process.

Suitable biodegradable materials include polylactic acid, polyglycolic acid (PGA), collagen or other connective proteins or natural materials, polycaprolactone, hylauric acid, adhesive proteins, copolymers of these materials as well as composites and combinations thereof and combinations of other biodegradable polymers. Other polymers that may be used include polyester and polycarbonate copolymers.

Examples of suitable metals include, but are not limited to, stainless steel, titanium, tantalum, platinum, tungsten, gold and alloys of any of the above- mentioned metals. Examples of suitable alloys include platinum-chromium alloys, platinum-iridium alloys, cobalt-chromium alloys including Elgiloy and Phynox, MP35N alloy and nickel-titanium alloys, for example, Nitinol. Examples of biodegradable alloys, such as magnesium alloys and zinc alloys, are disclosed in US 6854172 and US 2006/0052864.

The inventive expandable medical frameworks may be made of shape memory materials such as superelastic Nitinol or spring steel, or may be made of materials which are plastically deformable. In the case of shape memory materials, the expandable medical frameworks may be provided with a memorized shape and then deformed to a reduced diameter shape. The expandable medical framework may restore itself to its memorized shape upon being heated to a transition temperature and having any restraints removed therefrom.

In addition to or in place of the above materials, the inventive expandable medical frameworks may comprise ceramics.

The inventive expandable medical frameworks may include a coating or other portion of radiopaque material in one or more desired locations to allow for visualizing the device under X-rays. Examples of suitable locations for the radiopaque material include one or more ends of the framework and/or the middle of the framework. The expandable medical frameworks may likewise be provided with MRI sensitive markers to enhance MRI visibility under desired MRI imaging techniques.

The inventive expandable medical frameworks may be created by methods including laser or mechanical cutting or etching a design from a tubular stock or from a flat sheet. In the case of a flat sheet, the sheet may subsequently be rolled into tubular form. Optionally, the long edges of the sheet may be joined together, optionally by welding, to form a closed expandable medical framework. Alternatively, the edges may be left unsecured to one another. Any other suitable technique which is known in the art or which is subsequently developed may also be used to manufacture the inventive expandable medical frameworks disclosed herein.

The inventive expandable medical frameworks may be polished and cleaned as necessary using any suitable technique which is known in the art or which is subsequently developed.

Optionally, at least a portion of the expandable medical framework may be configured to include one or more mechanisms for the delivery of a therapeutic agent. Often the agent will be in the form of a coating or other layer (or layers) of material placed on a surface region of the expandable medical framework, which is adapted to be released at the site of the expandable medical framework's implantation or areas adjacent thereto.

A therapeutic agent may be a drug or other pharmaceutical product such as non-genetic agents, genetic agents, cellular material, etc. Some examples of suitable non-genetic therapeutic agents include but are not limited to: anti-thrombogenic agents such as heparin, heparin derivatives, vascular cell growth promoters, growth factor inhibitors, Paclitaxel, etc.

Some other examples of therapeutic agents include everolimus and sirolimus, their analogs and conjugates. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof. The therapeutic agent may be provided by itself or in conjunction with a polymer agent. The polymer agent may be a polystyrene - polyisobutylene-polystyrene triblock copolymer (SIBS), polyethylene oxide, silicone rubber and/or any other suitable substrate. The inventive expandable medical frameworks may include one or more of the above-mentioned therapeutic agents and/or polymers.

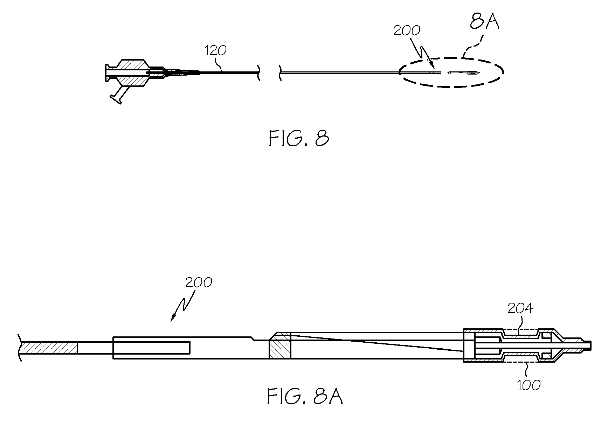

The expandable medical frameworks disclosed herein may be delivered to a desired bodily location via a catheter. Stent 100 is shown schematically, in Figure 8, being carried on catheter 200 including balloon 204 at the distal end of the catheter. In the case of balloon expandable medical frameworks including stents and stent-grafts, the catheter will include a balloon and the expandable medical framework will be disposed about the balloon. The balloon will typically be expanded once the expandable medical framework is delivered to a desired location in the body. This will result in expansion of the expandable medical framework. The balloon may then be deflated and the catheter repositioned within the body or withdrawn therefrom.

In the case of a mechanically expandable medical framework, including stents and stent-grafts, the catheter will include a mechanism capable of mechanically expanding the expandable medical framework. The mechanism will typically be expanded once the expandable medical framework is delivered to a desired location in the body. This will result in expansion of the expandable medical framework. The catheter may then be repositioned within the body or withdrawn therefrom.

In the case of a self-expanding expandable medical framework, including stents and stent-grafts, the catheter will typically include a sheath disposed about the expandable medical framework. Once the sheath is withdrawn or retracted from expandable medical framework, the expandable medical framework will self-expand. The catheter may then be repositioned within the body or withdrawn therefrom.

The following example is directed to the combination of an expandable medical framework and a catheter. The catheter may be any of those disclosed above or any other suitable catheter.

The invention is also directed to a catheter with any of the inventive expandable medical frameworks disclosed herein disposed at or near the distal end thereof. The expandable medical framework may be disposed about a balloon in the case of balloon expandable medical frameworks. The expandable medical framework may be disposed within a sheath in the case of self-expandable expandable medical frameworks.

The following example describes a method of delivering an expandable medical framework to a desired location in a body and methods of treating a portion of a body. The method involves providing a catheter carrying an expandable medical framework disclosed herein, using the catheter to delivering the expandable medical framework to the desired bodily location and deploying the expandable medical framework at the desired bodily location. Where the expandable medical framework is a balloon expandable stent, the stent, the balloon will be inflated at the desired bodily location and the stent thereby deployed.

Where the expandable medical framework is a self-expandable stent, a sheath covering the stent is retracted at the desired bodily location and the stent thereby deployed.

Following deployment, the catheter is withdrawn from the body.

The above disclosure is intended to be illustrative and not exhaustive. This description will suggest many variations and alternatives to one of ordinary skill in this art. The various elements shown in the individual figures and described above may be combined or modified for combination as desired. All these alternatives and variations are intended to be included within the scope of the claims where the term "comprising" means "including, but not limited to".

Further, the particular features presented in the dependent claims can be combined with each other in other manners within the scope of the invention such that the invention should be recognized as also specifically directed to other embodiments having any other possible combination of the features of the dependent claims. For instance, for purposes of claim publication, any dependent claim which follows should be taken as alternatively written in a multiple dependent form from all prior claims which possess all antecedents referenced in such dependent claim if such multiple dependent format is an accepted format within the jurisdiction (e.g. each claim depending directly from claim 1 should be alternatively taken as depending from all previous claims). In jurisdictions where multiple dependent claim formats are restricted, the following dependent claims should each be also taken as alternatively written in each singly dependent claim format which creates a dependency from a prior antecedent-possessing claim other than the specific claim listed in such dependent claim below. This completes the description of the invention. Those skilled in the art may recognize other equivalents to the specific embodiment described herein which equivalents are intended to be encompassed by the claims attached hereto.

## Claims

1. An expandable medical framework for implantation in a mammalian body comprising:
a plurality of serpentine bands (104; 104a; 104b) including intermediate serpentine bands (104b) and end serpentine bands (104a), the intermediate serpentine bands (104b) disposed between the end serpentine bands (104a), each serpentine band (104; 104a; 104b) comprising a plurality of alternating straight band struts (108) and turns (112); and
a plurality of connector columns, each connector column comprising a plurality of connector struts (120), each connector column disposed between and connecting two serpentine bands (104; 104a; 104b), each connector strut (120) connected at one end to a turn (112) of one serpentine band (104; 104a; 104b) and connected at the other end to a turn (112) of another serpentine band (104; 104a; 104b),
**characterized in that**:
the turns (112) of a serpentine band (104; 104a; 104b) comprising connected turns and unconnected turns, each connected turn having a connector strut (120) extending therefrom, each unconnected turn not having a connector strut (120) extending therefrom; each of the end serpentine bands (104a) connected to one of said intermediate serpentine bands (104b) via four or five connector struts (120) which are equally spaced about the circumference of the expandable medical framework, each two intermediate serpentine bands (104b) which are adjacent one another are connected one to the other via two connector struts (120), the two connector struts (120) being equally spaced about the circumference of the expandable medical framework.

2. The expandable medical framework of claim 1 in the form of a balloon-expandable stent.

3. The expandable medical framework of claim 1, wherein the connector struts (120) within a connector column are all oriented at the same angle relative to the longitudinal axis and the connector struts in a connector column adjacent thereto are all oppositely oriented relative to said angle.

4. The expandable medical framework of claim 3, wherein the straight band struts (120) have outer surfaces facing radially outward and inner surfaces facing radially inward, wherein the outer surfaces have rounded edges.

5. The expandable medical framework of claim 4, wherein the unconnected turns (112) each have an outer radius as measured on the outside of the unconnected turn, wherein the outer radius is between 0.0035 inches and 0.0050 inches (0.089 mm and 0.127 mm).

6. The expandable medical framework of claim 5 having a thickness as measured in a radial direction of between 0.0025 inches and 0.0034 inches (0.0635 mm and 0.08636 mm).

7. The expandable medical framework of claim 6 wherein the angle of the connector struts (120) in the connector column alternates between 35 degrees relative to a longitudinal axis bisecting the connected turn (112) from which the connector strut extends and 50 degrees relative to a longitudinal axis bisecting the connected turn (112) from which the connector strut extends (120).

8. The expandable medical framework of claim 7, wherein each serpentine band (104; 104a; 104b) has sixteen struts (108) and wherein there only three turns (112) disposed between adjacent connector struts (120) connecting an end serpentine band (104a) to an intermediate serpentine band (104b).

9. The expandable medical framework of claim 7, wherein each serpentine band (104; 104a; 104b)has twenty struts and wherein there are only three turns (112) disposed between adjacent connector struts (120) connecting an end serpentine band (104a) to an intermediate serpentine band (104b).

## Patentansprüche

1. Expandierbarer medizinischer Rahmen zur Implantierung in einem Säugetierkörper, umfassend:
eine Vielzahl von serpentinenförmigen Bändern (104; 104a; 104b), einschließlich serpentinenförmiger Zwischenbänder (104b) und serpentinenförmiger Endbänder (104a), wobei die serpentinenförmigen Zwischenbänder (104b) zwischen den serpentinenförmigen Endbändern (104a) angeordnet sind, wobei jedes serpentinenförmige Band (104; 104a; 104b) eine Vielzahl von abwechselnden geraden Bandstreben (108) und Wendungen (112) umfasst, und eine Vielzahl von Verbinderkolonnen, wobei jede Verbinderkolonne eine Vielzahl von Verbinderstreben (120) umfasst, wobei jede Verbinderkolonne zwischen zwei serpentinenförmigen Bändern (104; 104a; 104b) angeordnet ist und diese verbindet, wobei jede Verbinderstrebe (120) an einem Ende mit einer Wendung (112) eines serpentinenförmigen Bands (104; 104a; 104b) verbunden ist und am anderen Ende mit einer Wendung (112) eines anderen serpentinenförmigen Bands (104; 104a; 104b) verbunden ist,
**dadurch gekennzeichnet, dass**
die Wendungen (112) eines serpentinenförmigen Bands (104; 104a; 104b) verbundene Wendungen und unverbundene Wendungen umfassen, wobei jede verbundene Wendung eine sich davon erstreckende Verbinderstrebe (120) hat und jede unverbundene Wendung keine sich davon erstreckende Verbinderstrebe (120) hat, jedes der serpentinenförmigen Endbänder (104a) über vier oder fünf Verbinderstreben (120), die gleichmäßig um den Umfang des expandierbaren medizinischen Rahmens herum beabstandet sind, mit einem der serpentinenförmigen Zwischenbänder (104b) verbunden ist und jeweils zwei serpentinenförmige Zwischenbänder (104b), die einander benachbart sind, über zwei Verbinderstreben (120) miteinander verbunden sind, wobei die beiden Verbinderstreben (120) gleichmäßig um den Umfang des expandierbaren medizinischen Rahmens herum beabstandet sind.

2. Expandierbarer medizinischer Rahmen nach Anspruch 1 in der Form eines ballon-expandierbaren Stents.

3. Expandierbarer medizinischer Rahmen nach Anspruch 1, wobei die Verbinderstreben (120) in einer Verbinderkolonne alle im selben Winkel bezüglich der Längsachse ausgerichtet sind und die Verbinderstreben in einer benachbarten Verbinderkolonne alle diesem Winkel entgegengesetzt ausgerichtet sind.

4. Expandierbarer medizinischer Rahmen nach Anspruch 3, wobei die geraden Bandstreben (120) Außenflächen, die radial nach außen weisen, und Innenflächen, die radial nach innen weisen, haben, wobei die Außenflächen abgerundete Ränder haben.

5. Expandierbarer medizinischer Rahmen nach Anspruch 4, wobei die unverbundenen Wendungen (112) jeweils einen äußeren Radius haben, wie außen an der unverbundenen Wendung gemessen, wobei der äußere Radius zwischen 0,089 mm und 0,127 mm (0,0035 Zoll und 0,0050 Zoll) beträgt.

6. Expandierbarer medizinischer Rahmen nach Anspruch 5, mit einer Dicke, wie in einer radialen Richtung gemessen, von zwischen 0,0635 mm und 0,08636 mm (0,0025 Zoll und 0,0034 Zoll).

7. Expandierbarer medizinischer Rahmen nach Anspruch 6, wobei der Winkel der Verbinderstreben (120) in der Verbinderkolonne zwischen 35 Grad bezüglich einer Längsachse, die die verbundene Wendung (112) schneidet, von der sich die Verbinderstrebe erstreckt, und 50 Grad bezüglich einer Längsachse, die die verbundene Wendung (112) schneidet, von der sich die Verbinderstrebe (120) erstreckt, wechselt.

8. Expandierbarer medizinischer Rahmen nach Anspruch 7, wobei jedes serpentinenförmige Band (104; 104a; 104b) sechzehn Streben (108) hat und wobei nur drei Wendungen (112) zwischen benachbarten Verbinderstreben (120), die ein serpentinenförmiges Endband (104a) mit einem serpentinenförmigen Zwischenband (104b) verbinden, angeordnet sind.

9. Expandierbarer medizinischer Rahmen nach Anspruch 7, wobei jedes serpentinenförmige Band (104; 104a; 104b) zwanzig Streben hat und wobei nur drei Wendungen (112) zwischen benachbarten Verbinderstreben (120), die ein serpentinenförmiges Endband (104a) mit einem serpentinenförmigen Zwischenband (104b) verbinden, angeordnet sind.

## Revendications

1. Cadre médical extensible destiné à être implanté dans le corps d'un mammifère comportant :
une pluralité de bandes (104 ; 104a ; 104b) sinueuses comprenant des bandes (104b) sinueuses intermédiaires et des bandes (104a) sinueuses d'extrémité, les bandes (104b) sinueuses intermédiaires étant disposées entre les bandes (104a) sinueuses d'extrémité, chaque bande (104 ; 104a ; 104b) sinueuse comportant une pluralité d'entretoises (108) de bandes droites et de coudes (112) alternés ; et
une pluralité de colonnes de liaison, chaque colonne de liaison comportant une pluralité d'entretoises (120) de liaison, chaque colonne de liaison étant disposée entre et reliant deux bandes (104 ; 104a ; 104b) sinueuses, chaque entretoise (120) de liaison étant reliée au niveau d'une extrémité à un coude (112) d'une bande (104 ; 104a ; 104b) sinueuse et reliée au niveau de l'autre extrémité à un coude (112) d'une autre bande (104 ; 104a ; 104b) sinueuse,
**caractérisé en ce que** :
les coudes (112) d'une bande (104 ; 104a ; 104b) sinueuse comportant des coudes reliés et des coudes non reliés, chaque coude relié présentant une entretoise (120) de liaison qui s'étend depuis celui-ci, chaque coude non relié ne présentant pas d'entretoise (120) de liaison qui s'étend depuis celui-ci ; chacune des bandes (104a) sinueuses d'extrémité reliée à l'une desdites bandes (104b) sinueuses intermédiaires par le biais de quatre ou cinq entretoises (120) de liaison qui sont espacées de manière équidistante autour de la circonférence du cadre médical extensible, toutes les deux bandes (104b) sinueuses intermédiaires qui sont adjacentes l'une à l'autre sont reliées l'une à l'autre par le biais de deux entretoises (120) de liaison, les deux entretoises (120) de liaison étant espacées de manière équidistante autour de la circonférence du cadre médical extensible.

2. Cadre médical extensible selon la revendication 1 sous forme d'endoprothèse extensible par ballonnet.

3. Cadre médical extensible selon la revendication 1, dans lequel les entretoises (120) de liaison à l'intérieur d'une colonne de liaison sont toutes orientées selon le même angle par rapport à l'axe longitudinal et les entretoises de liaison dans une colonne de liaison adjacente à celles-ci sont toutes orientées de manière opposée par rapport audit angle.

4. Cadre médical extensible selon la revendication 3, dans lequel les entretoises (120) de bandes droites présentent des surfaces extérieures tournées radialement vers l'extérieur et des surfaces intérieures tournées radialement vers l'intérieur, les surfaces extérieures présentant des bords arrondis.

5. Cadre médical extensible selon la revendication 4, dans lequel les coudes (112) non reliés ont chacun un rayon extérieur tel que mesuré sur l'extérieur du coude non relié, le rayon extérieur étant compris entre 0,0035 et 0,0050 pouce (0,089 et 0,127 mm).

6. Cadre médical extensible selon la revendication 5 présentant une épaisseur telle que mesurée dans une direction radiale comprise entre 0,0025 et 0,0034 pouce (0,0635 et 0,08636 mm).

7. Cadre médical extensible selon la revendication 6 dans lequel l'angle des entretoises (120) de liaison dans la colonne de liaison alterne entre 35 degrés par rapport à un axe longitudinal coupant en deux le coude (112) relié depuis lequel s'étend l'entretoise de liaison et 50 degrés par rapport à un axe longitudinal coupant en deux le coude (112) relié depuis lequel s'étend l'entretoise de liaison (120).

8. Cadre médical extensible selon la revendication 7, dans lequel chaque bande (104 ; 104a ; 104b) sinueuse présente seize entretoises (108) et dans lequel il n'y a que trois coudes (112) disposés entre les entretoises (120) de liaison adjacentes reliant une bande (104a) sinueuse d'extrémité à une bande (104b) sinueuse intermédiaire.

9. Cadre médical extensible selon la revendication 7, dans lequel chaque bande (104 ; 104a ; 104b) sinueuse présente vingt entretoises et dans lequel il n'y a que trois coudes (112) disposés entre les entretoises (120) de liaison adjacentes reliant une bande (104a) sinueuse d'extrémité à une bande (104b) sinueuse intermédiaire.
